# EUROPEAN PATENT APPLICATION

(11) **EP 1 149 601 A1**
(43) Date of publication of application: **31.10.2001**
(21) Application number: 01110286.0
(22) Date of filing: 26.04.2001
(51) Int. Cl.: A61M 5/32, A61B 19/02

(54) **Sharps disposal assembly having improved receiving aperture**

(30) Priority: 27.04.2000 US 559042
(71) Applicant: Becton, Dickinson and Company, Franklin Lakes, New Jersey 07417 (US)
(72) Inventor: Bickel, Christopher R., Parsippany, New Jersey 07054 (US); Seifert, Kevin J., Kinnelon, New Jersey 07405 (US); Vincenti, David F., West Caldwell, New Jersey 07006 (US)
(74) Representative: von Kreisler, Alek, Dipl.-Chem.

(57) **Abstract**

A sharps disposal assembly (10) has a receptacle (12) and lid (14) therefore. A plate portion of the lid (14) has an opening (16) therethrough which includes a port segment (18) for receiving large components of a used medical article and an unwinder segment (20) continuous with the port assembly.

## Description

1. Field of the Invention: This invention relates to medical devices and more particularly relates to blood sampling devices and safe disposal thereof.

2. Background of the Invention: Many medical articles used in hospitals and clinics are designed for one-time use. Articles which have sharp points, cutting edges and the like are collectively known as sharps, and many disclosures of special equipment and procedures have been proposed to minimize the danger of injury, such as needle stick, to personnel involved in the use and disposal of these articles. Safe handling and disposal is particularly important, since a sharp is often used in a procedure, such as blood sampling, and as a result may be contaminated with a potentially infectious agent.

Many designs of disposal equipment for sharps have been proposed. Most include a storage container having a lid with locking closure features and several openings through the lid for access to the interior of the container. One of the openings has specialized structure associated therewith, known in the art as the unwinder, for detaching a sharp, such as a needle-hub unit, from a tube holder or syringe.

In addition to the unwinder, disposal assemblies heretofore disclosed or used in medical practice have a second large opening through the lid, generally referred to as the port opening, for disposing of an article having a component which is too large or unwieldy to insert through the unwinder opening. Typical disposal assemblies having an opening for unwinding a sharp and a separate opening for receiving a larger component to be discarded are disclosed in US Patent No. 5,107,990 to Wicherski et al. and 5,092,462 to Sagstetteret al.

Prior art assemblies having one opening through the lid for unwinding and another separate opening for disposal of large components such as syringe barrels, tube holders, winged needles and the like require multiple manual operations by the technician and accordingly put the technician at increased risk of accident. In fact, some articles cannot be discarded with prior art assemblies because a large component of the article interferes with unwinding a second component. For example, disposal of a used blood collection set requires insertion of a large component (a winged needle) into the container before the needle hub unit can be mated to the unwinder. Thus, a technician faced with disposal of a used blood collection set with a prior art assembly must hold the used needle by the wings (i.e., with the used needles exposed), manually unwind the holder from the hub at the distal end of the tubing, and then insert the needle-tubing-hub unit into the container through the port opening all the while avoiding contact with the exposed used insertion and delivery needles.

There is a need in the art for a disposal assembly capable of unwinding a needle-hub unit and at the same time providing ready disposal of a larger component of a blood collection device without manual intervention. The present invention addresses this need.

### SUMMARY OF THE INVENTION

A sharps disposal assembly includes a receptacle and a lid for the receptacle having an opening therethrough. The opening has a large port segment and a substantially V-shaped unwinder segment continuous with the port segment. The unwinder segment may include any structure suitable for unwinding a sharp from an associated structure, such as a needle holder.

The combined port and unwinder segments enable a technician to unwind a sharp from any associated component, regardless of its size, and insert both into the receptacle without any manual manipulation. The assembly is particularly useful for disposal of a conventional blood collection set, wherein a technician, grasping only the holder, can insert the contaminated winged needle and tubing through the large port opening, and, still with no manual manipulation, can advance the collar of the holder until the hub engages the unwinder. After unwinding, the complete unit simply drops into the receptacle. The complete disposal operation requires no danger of contact between the technician and the used needles. With a conventional assembly having a separate port and unwinder, the winged needle prevents the needle-hub from entering the unwinder.

### BRIEF DESCRIPTION OF THE DRAWINGS

Fig 1 is a perspective view of a sharps disposal assembly of the invention showing the combined port and unwinder openings;
Fig 2 is a perspective view of the assembly of Fig 1 being used for disposal of a syringe;
Fig 3 is a perspective view of the assembly of Fig 1 being used for disposal of a blood collection set; and
Fig 4 is a perspective view of an alternative embodiment of the invention.

### DETAILED DESCRIPTION

While this invention is satisfied by embodiments in many different forms, there will herein be described in detail embodiments of the invention with the understanding that the present disclosure is to be considered as exemplary of the principles of the invention and is not intended to limit the invention to the embodiments illustrated and described. The scope of the invention will be measured by the appended claims and their equivalents.

The sharps disposal assembly of the present invention improves over prior art assemblies by reducing handling during disposal of any medical article having a large component and a component for unwinding. Thus, for example, the invention contemplates disposal of a syringe having a needle-hub unit to be unwound from a hub on the barrel of the syringe. The assembly of the invention is particularly directed to disposal of a conventional blood collection set, as described in detail below.

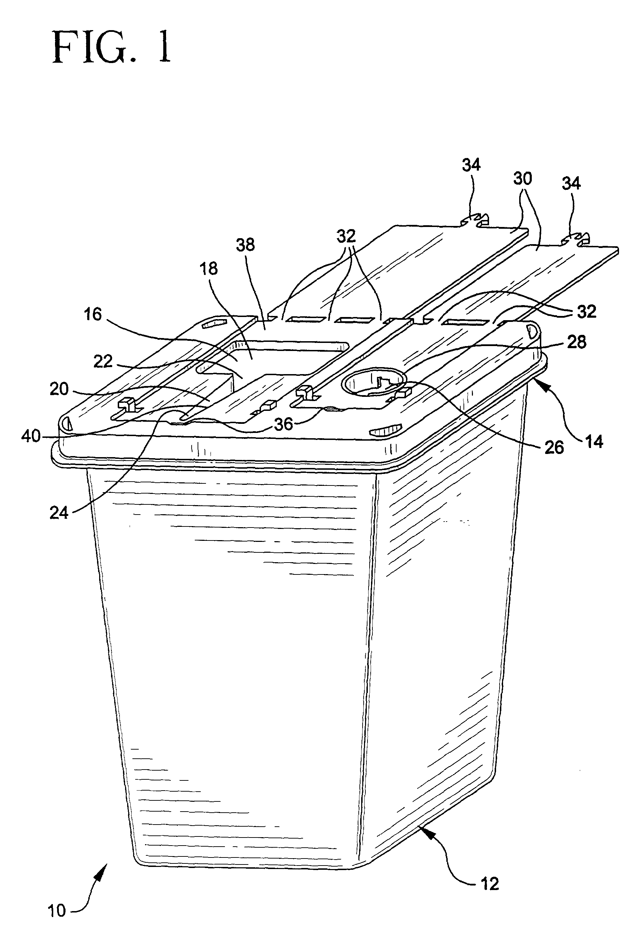

Adverting now to the drawings, Fig 1 illustrates a sharps disposal assembly 10 including a receptacle 12 and a lid 14. Lid 14 has opening 16 therethrough providing access to the interior volume of receptacle 12. Opening 16 has a large port segment 18, shown to have the preferred rectangular shape, although it is understood that any geometrical configuration would be suitable. A second segment of opening 16 is an unwinder segment 20 which is continuous with the port segment. Segment 20 is shown to be of a preferred slot or V-shape, but may have any other shape suitable for unwinding.

Unwinder segment 20 has wide end 22 tapering to narrow end 24. Annular opening 26 has insert 28 therein, expressly dimensioned to engage a SAFETY-GARD™ tube holder as sold by Becton, Dickinson and Company and fully disclosed in the aforementioned US Patent No. 5,092,462.

Flaps 30 are affixed to lid 14 by integral tabs 32 and have integral closure tabs 34 dimensioned for insertion into slots 36 of a plate portion 38 of lid 14 to cover the openings.

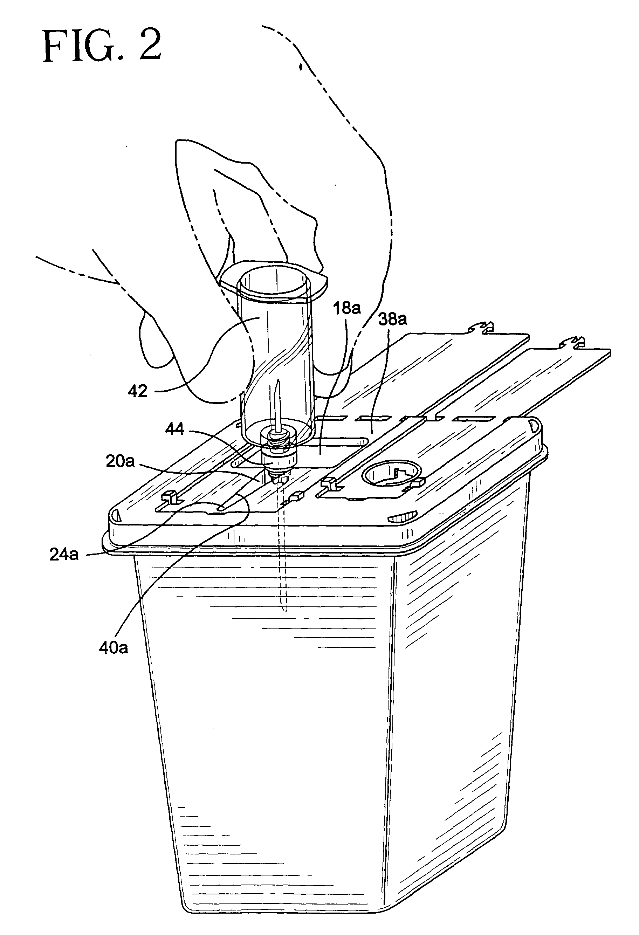

Unwinder segment 20 of opening 16 has a wall 40 therearound. Wall 40 may include any structure as known in the art, such as a notch, for immobilizing a sharp in a substantially stable position while detaching or unwinding the sharp from an associated component, such as a hub. Fig 2 illustrates a syringe barrel 42 having a needle-hub unit 44 threaded thereto and inserted into port opening 18a and ready to be advanced toward narrow end 24a of segment 20a until wall 40a, or a notch therein, grips the hub unit 44. Conventional unwinding may then be performed so that the needle hub unit falls into the receptacle and the syringe barrel may be retained for reuse. Port segment 18a is large enough to receive the barrel if this is also to be discarded.

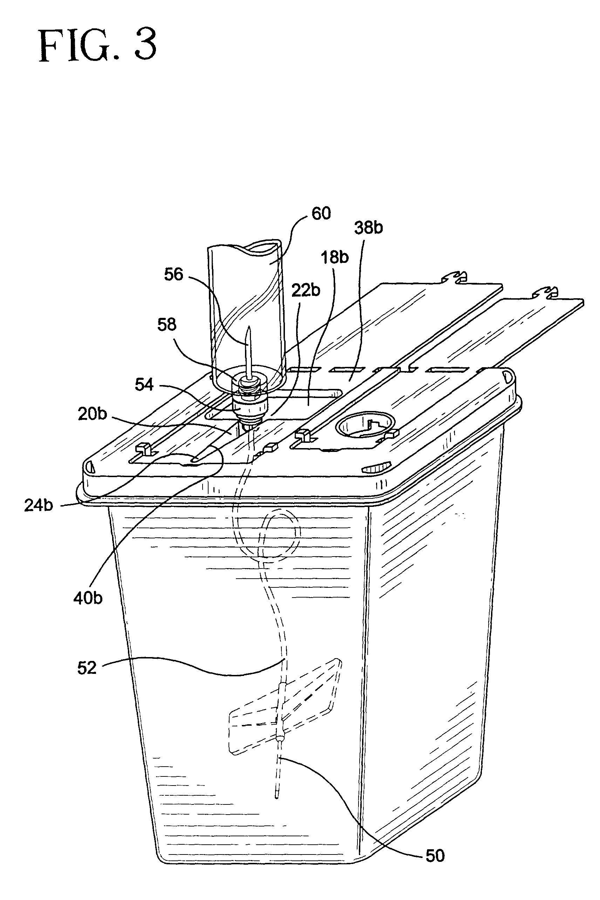

Fig 3 illustrates a preferred use of the combined port-unwinder of the invention for disposal of a conventional blood collection set. A used collection set to be discarded includes a conventional winged needle 50 for skin penetration affixed to a tubing 52 having a threaded hub 54 and delivery needle 56 on the distal end thereof. Threaded hub 54 may have a conventional rib (not shown in the drawing) thereon, and is affixed to a mating threaded collar 58 on a conventional tube holder 60.

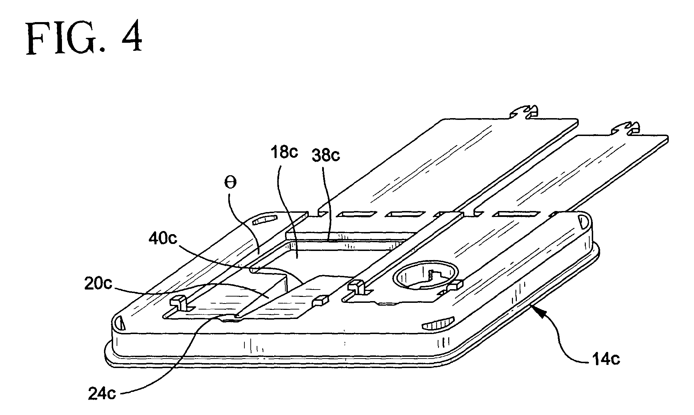

In another embodiment of the invention, illustrated in Fig 4, plate portion 38c of lid 14c may be sloped at an angle theta from the plane of lid 14c. Angle theta may be about 5-20°, preferably 10-15°, so that narrow unwinder end 24c is higher than port segment 18c. This configuration causes a needle-hub unit, after unwinding, to slide down the slope of wall 40c and thereby aids in overcoming hangup of the unit on the wall so it can drop freely into the container.

In use, winged needle 50 and tubing 52 are inserted, while grasping the tube holder only, through port segment 18b until collar 58 is positioned adjacent to wide end 22b of unwinder segment 20b. Tube holder 60 is then advanced toward narrow end 24b of the unwinder segment until threaded hub 54 engages wall 40b of the unwinder segment. Counterclockwise rotation of the tube holder then unwinds the threaded collar of the holder from the threads on the hub so the winged needle and tubinghub unit affixed thereto fall into the receptacle.

## Claims

1. An assembly for receiving and disposing of a sharp comprising:
a) a substantially rigid storage receptacle;
b) a lid for said receptacle comprising plate means defining an opening therethrough, said opening having a port segment and an unwinder segment continuous with said port segment; and
c) a wall around said unwinder segment.

2. The assembly of Claim 1 wherein said port segment is rectangular and dimensioned to receive a large medical article.

3. The assembly of Claim 2 wherein said large medical article may be a syringe, needle holder or blood collection set.

4. The assembly of Claim 1 wherein said unwinder segment is V shaped.

5. The assembly of Claim 1 wherein said wall further comprises unwinding means.

6. The assembly of Claim 5 wherein said unwinding means is a notch in said wall.

7. The assembly of Claim 1 wherein said plate means is sloped at an angle to said lid.

8. The assembly of Claim 1 wherein said lid further comprises closure means affixed thereto for covering said openings.

9. The assembly of Claim 8 wherein said closure means is a flap having a tab to affix said flap to said lid.

10. An assembly for receiving and disposing of a blood collection set comprising:
a) a substantially rigid storage receptacle;
b) a lid for said receptacle, said lid having a plate segment defining an opening, said opening having a port segment and an unwinder segment continuous with said port segment;
c) a wall around said unwinder segment, said unwinder having structure for engaging a rib on a needle-hub unit threaded to a needle holder; and
d) a flap affixed to said lid having a tab thereon for affixing said flap to said lid.
